# EUROPÄISCHE PATENTSCHRIFT

(11) **EP 1 034 768 B1**
(45) Veröffentlichungstag und Bekanntmachung des Hinweises auf die Patenterteilung: **07.08.2002**
(21) Anmeldenummer: 00102772.1
(22) Anmeldetag: 10.02.2000
(51) Int. Cl.: A61G 13/00

(54) **Einrichtung mit einer auf einem Ständer abnehmbar gelagerten Operations-Tischplatte**
Device comprising a support and a removable operating table top thereon
Dispositif avec un plateau de table d'opération amovible d'un support

(30) Priorität: 09.03.1999 DE 19910289
(43) Veröffentlichungstag der Anmeldung: 13.09.2000
(73) Patentinhaber: TRUMPF Kreuzer Medizin Systeme GmbH & Co. KG, 82178 Puchheim (DE)
(72) Erfinder: Kreuzer, Friedhelm, 81247 M-nchen (DE)
(74) Vertreter: Prüfer, Lutz H., Dipl.-Phys.

(56) Entgegenhaltungen:
- DE-A- 3 704 860
- US-A- 3 757 420
- US-A- 5 735 278
- US-A- 5 842 987

## Beschreibung

Die Erfindung betrifft eine Einrichtung mit einer auf einem Ständer eines Operationstisches abnehmbar gelagerten Operationstischplatte und einer Anästhesiestation und einer in einem Abstand von dem Operationstisch angeordneten Untersuchungsstation.

Aus US 5 842 987 ist eine Einrichtung bekannt, bei der Patienten mittels eines abnehmbaren Tischaufsatzes zwischen verschiedenen in einem Abstand voneinander angeordneten Untersuchungsstationen transferiert werden. Dabei kann der Tischaufsatz auf den jeweiligen Behandlungstischen montiert werden. In der Druckschrift wird auch ein Operationsraum vorgesehen, jedoch muß der Patient für einen chirurgischen Eingriff vom Tischaufsatz auf den Operationstisch verlagert werden.

Bei Schwerverletzten bedeutet jedoch das Umbetten zum Zweck des Transportes zwischen verschiedenen Stationen jeweils ein erhebliches Risiko. Um dieses Risiko zu mindern wurde eine transportable Operationstischplatte geschaffen, die von ihrer Struktur so ausgebildet ist, daß sie gleichzeitig auch als Liege verwendet werden kann, wenn der Patient beispielsweise in einer Untersuchungstation zu untersuchen ist.

Aufgabe der Erfindung ist es, eine Einrichtung zu schaffen, die die Patientenversorgung bei gleichzeitiger Erhöhung der Sicherheit für Anwender und Patient noch weiter verbessert.

Diese Aufgabe wird durch die in Patentanspruch 1 gekennzeichnete Einrichtung gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren.

Von den Figuren zeigen:
- Fig. 1: eine Seitenansicht der Einrichtung in Operationsstellung;
- Fig. 2: eine Draufsicht auf die in Fig. 1 gezeigte Einrichtung;
- Fig. 3: eine Seitenansicht in der Tischaufnahme-Position;
- Fig. 4: eine Draufsicht der Einrichtung nach Fig. 3;
- Fig. 5: eine Seitenansicht in Ablageposition;
- Fig. 6: eine Seitenansicht in Untersuchungsposition.

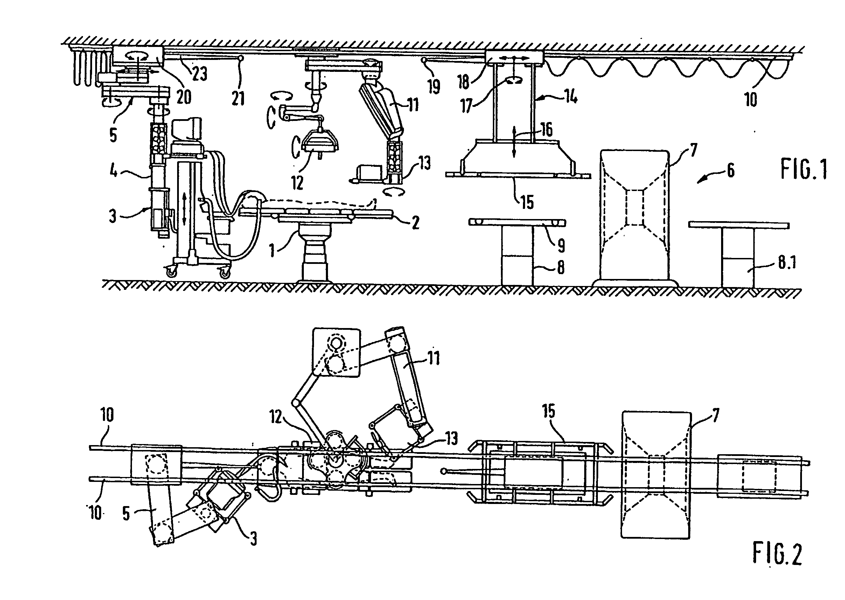

Wie am besten aus Fig. 1 und 2 zu ersehen ist, umfaßt die Einrichtung einen Operationstisch mit einem Ständer 1 mit einem Aufnahmerahmen und einer darauf abnehmbar gelagerten Operationstischplatte 2. Am Kopfende des Operationstisches ist eine Anästhesiestation 3 mit den üblichen Geräten wie Monitor, Narkosegerät und Infusionssystem vorgesehen, die an einer Säule 4 integriert sind und von einem Arm eines Deckenstatives 5 gehalten ist.

Auf der dem Kopfende abgewandten Seite des Operationstisches ist in einem Abstand von dem Operationstisch eine Tomographiestation 6 angeordnet. Diese umfaßt den eigentlichen Tomographen 7 und auf der dem Operationstisch zuwandten Eingangsseite einen Ständer 8 und an der Ausgangsseite einen Ständer 8.1 mit jeweils einem dem Tragrahmen des Ständers 1 entsprechenden Tragrahmen 9 zum Aufnehmen der Operationstischplatte 2.

An der Decke des Operationsraumes ist eine als Schienenpaar ausgebildete Schiene 10 vorgesehen, die auch als einzelne Schiene ausgebildet sein kann. Seitlich von dem Operationstisch und seitlich von der Schiene ist eine Chirurgiestation 11 mit einer schwenkbaren OP-Leuchte 12 und einem Stativkopf 13 zur Aufnahme von Chirugiegeräten und Zusatzgeräten angeordnet, deren Arbeitspositionen am besten aus Fig. 2 ersichtlich sind.

Wie am besten aus Fig. 1 ersichtlich ist, ist an der Schiene 10 auf der dem Deckenstativ 5 abgewandten und der Tomographiestation 6 zugewandten Seiten ein Operationstischplatten-Deckentransporter 14 vorgesehen. Dieser weist einen in seiner Höhe in Richtung des Pfeiles 16 verfahrbaren und durch den Pfeil 17 angedeutet drehbar gelagerten Aufnahmerahmen 15 sowie das eigentliche Fahrgestell 18 auf. An dem Fahrgestell 18 ist ein sich in Richtung zu dem Deckenstativ 5 hin erstreckendes erstes Kupplungselement 19 angeordnet. Das Deckenstativ 5 weist ein dem Fahrgestell entsprechendes zweites Fahrgestell 20 auf. Dieses ist mit einem dem ersten Kupplungselement 19 zugewandten zweiten Kupplungselement 21 versehen, welches so ausgebildet ist, daß es mit dem ersten Kupplungselement 19 in Eingriff bringbar ist.

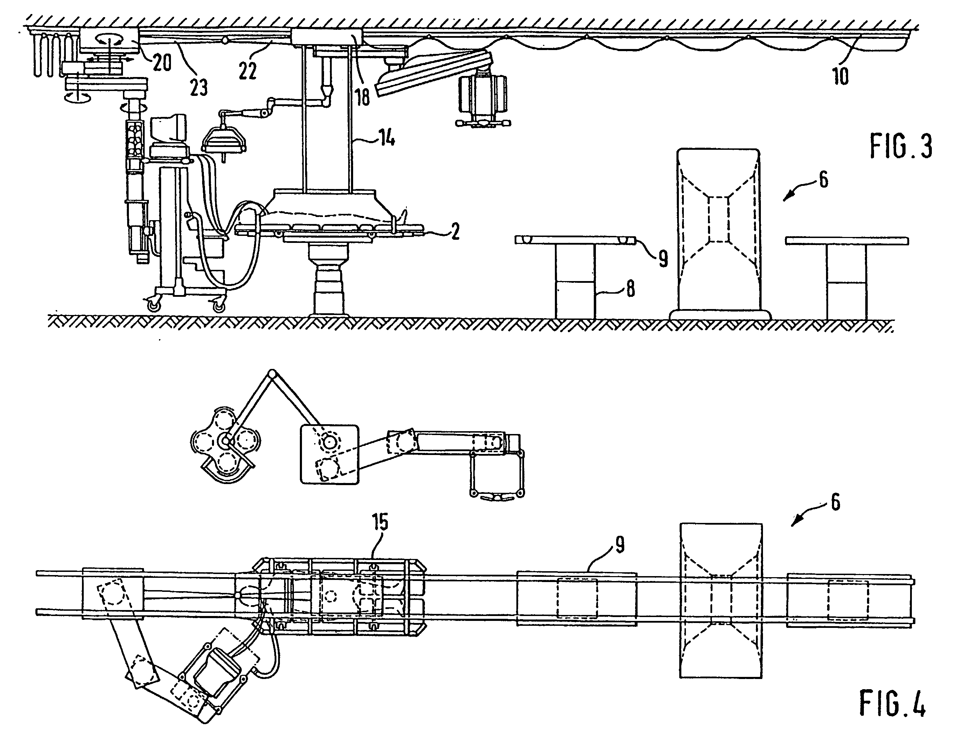

Wie am besten aus Fig. 3 und 4 ersichtlich ist, ist der Deckentransporter 14 so in Richtung zu dem Ständer 1 verfahrbar, daß er die Operationstischplatte 2 aufnehmen kann. Dabei befindet sich, wie man in Fig. 4 sieht, die Chirurgiestation 11 mit einer schwenkbaren OP-Leuchte 12 und einem Stativkopf 13 zur Aufnahme von Chirugiegeräten und Zusatzgeräten in einer Transferposition. Wie ferner insbesondere aus Fig. 3 ersichtlich ist, sind das erste und zweite Kupplungselement 19, 21 über jeweilige Arme 22, 23 mit dem Fahrgestell 18 bzw. dem zweiten Fahrgestell 20 verbunden. Die Länge der Arme 22, 23 ist dabei so bemessen, daß das erste und zweite Kupplungselement in der in Fig. 3 gezeigten Position genau dann in Eingriff gelangen kann, wenn der Deckentransporter 14 die Position einnimmt, in der er die Operationstischplatte 2 aufnehmen kann. Die Positionsbestimmung erfolgt durch einen an der Schiene 10 justierbar angebrachten Schaltnocken, welche über Endschalter eine im Laufwagen 18 eingebaute Magnetbremse einschaltet. Durch die verbundenen Kupplungselemente wird gleichzeitig die Anästhesiestation an der Bewegung weg vom Patienten gehindert, was Schlauch/Kabeltrennung damit Lebensgefahr, ausschließt.

Der Deckentransporter 14 ist so ausgebildet, daß ein Aufnahmerahmen 15 von Hand mit der Operationstischplatte 2 zum Aufnehmen derselben verbindbar ist. Der Deckentransporter 14 weist einen motorischen Antrieb zur vertikalen Höhenverstellung des Aufnahmerahmens 15 auf. Dieser Antrieb ist über einen Sensor gesteuert, der so ausgebildet ist, daß der Sensor erfaßt, ob das erste und zweite Kupplungselement 19, 21 miteinander gekoppelt sind oder nicht. Dabei ist die Steuerung so ausgebildet, daß der Motor die Höhe des Aufnahmerahmens 15 nur dann verändern kann, wenn eine Kopplung der beiden Kupplungselemente 19, 21 festgestellt wird.

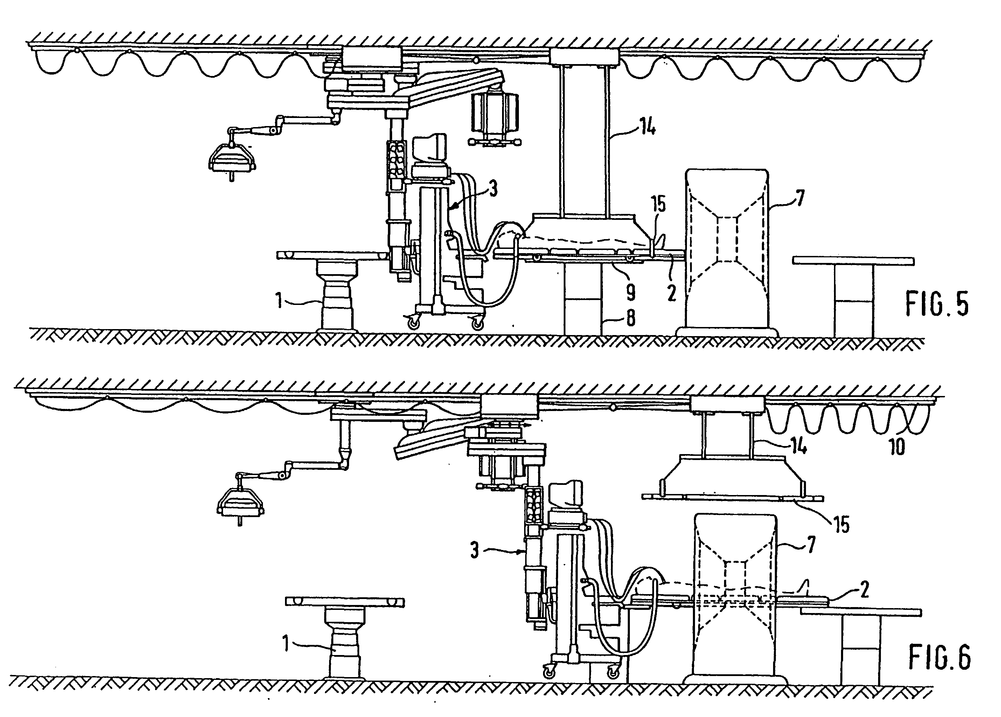

Wie aus den Figuren ersichtlich ist, ist der Deckentransporter 14 zwischen der in Fig. 3 gezeigten Aufnahme-/Ablageposition zur Aufnahme/Ablage der Operationstischplatte 2 vom Operationstisch zu einer in Fig. 5 gezeigten Ablage-/Aufnahmeposition über dem Ständer 8 und einer in Fig. 6 gezeigten dritten Parkposition hin- und herbewegbar. Mit Hilfe des Deckentransporters 14 und des Aufnahmerahmens 15 ist die Operationstischplatte 2 zwischen der in Fig. 1 gezeigten Operationstisch-Position und der in Fig. 5 gezeigten Tomographieständer-Position hin- und herfahrbar. Die Bewegung kann je nach Bedarf entweder von Hand oder durch einen im Laufwagen 18 des Deckentransporters 14 eingebauten Elektromotor bewirkt werden. Durch die oben beschriebene Zwangskupplung erfolgt ein Verfahren der Operationstischplatte stets zusammen mit der angekoppelten Anästhesiestation 3 zur Versorgung des Patienten. In der in Fig. 5 dargestellten Position wird die Bewegung durch einen zweiten an der Schiene 10 angebrachten Schaltnocken gestoppt; bei Handbetrieb wird die Magnetbremse alleine eingeschaltet, bei Motorbetrieb der Motor aus- und die Magnetbremse eingeschaltet, gleichzeitig die Abwärtsbewegung des Aufnahmerahmens freigegeben, die von Hand ausgelöst wird. Nach Ablage der Operationstichplatte 2 auf und vollzogene Verbindung mit Tragrahmen 9 wird die Aufwärtsbewegung des Aufnahmerahmens 25 freigegeben, die wiederum von Hand ausgelöst wird. Nach Erreichen der in Fig. 6 dargestellten Höhenposition, welche eine Kollision zwischen Aufnahmerahmen 15 und Tomograph 7 auschließt, geben Sensoren den Beginn der Untersuchung frei, die wegen Strahlengefahr außerhalb des Raumes ausgelöst wird. Nach dem Start wird die Operationstischplatte 2 in bekannter Weise mit dem darauf liegenden Patienten in die Öffnung des Tomographen 7 bewegt. Zwecks Erhaltung der zu Narkosebeginn fixierten Lage der Schläuche/Kabel zwischen Patient und Anästhesiestation zieht synchron mit der Operationstischplatte 2 der motorisch betriebene Transporter 14 die Anästhesiestation 3 bis zur in Fig. 6 gezeigten Position. Dort wird über einen dritten an der Schiene 10 angebrachten Schaltnocken der Motor ausgeschaltet und der Laufwagen 18 mittels Magnetbremse sicher abgebremst. Nach der Untersuchung erfolgen die Bewegungsabläufe in umgekehrter Richtung, wobei eine Entkupplung zwischen dem ersten Kupplungselement 19 und dem zweiten Kupplungselement 21 erst wieder in der in Fig. 3 gezeigten Aufnahme-/Ablageposition möglich ist.

In dem obigen Ausführungsbeispiel ist die Einrichtung zum Transport der Operationstischplatte 2 zusammen mit der Anästhesiestation zwischen dem Operationstisch und einem Tomographen beschrieben. Die selbe Einrichtung kann auch zum Transport zwischen dem Operationstisch und einer anderen Arbeits- bzw. Untersuchungsstation in einem Operationssaal verwendet werden.

Es ist ebenfalls möglich, die vom Ständer (1) abnehmbare Operationstischplatte (2), die mit dem Aufnahmerahmen (15) verbindbar ist, um zur Untersuchungsstation (7) transportiert zu werden, durch ein Tragetuch zu ersetzen. In diesem Falle befindet sich das Tragetuch zwischen dem darauf liegenden Patienten und dem entsprechend mit einer Tischplatte ausgerüsteten Ständer. Zum Transport wird dann das Tragetuch an dem entsprechend gestalteten Aufnahmerahmen befestigt.

## Patentansprüche

1. Einrichtung mit einer auf einem Ständer(1) abnehmbar gelagerten Operationstischplatte (2) und einer Anästhesie-Station (3) und einer in einem Abstand von dem Operationstisch angeordneten Untersuchungsstation (7) mit einem Ständer (8) zum Aufnehmen der Operationstischplatte (2), **gekennzeichnet durch** eine sich über den Operationstisch und die Untersuchungsstation (7) ersteckende Deckenschiene (10), eine von der Schiene (10) gehaltene und auf dieser verfahrbare Transporteinrichtung (14) und ein die Anästhesie-Station (3) tragendes, von der Schiene (10) gehaltenes und auf dieser verfahrbares Deckenstativ (5),
wobei Transporteinrichtung (14) und Deckenstativ (5) jeweils miteinander Wirkende Kupplungsteile (19,21) aufweisen, welche Kuppelbar sind, sobald die Transporteinrichtung (14) in eine erste Position zum Aufnehmen der Operationstischplatte (2) gelangt.

2. Einrichtung nach Anspruch 1, **dadurch gekennzeichnet, daß** Deckenstativ (5) und die damit gekoppelte Transporteinrichtung (14) zu einer zweiten Position fahrbar sind, in der die Operationstischplatte (2) auf dem Aufnahmeständer der Untersuchungsstation (7) absetzbar ist.

3. Einrichtung nach Anspruch 1 oder 2, **dadurch gekennzeichnet, daß** Deckenstativ (5) mit der damit angekoppelten Transporteinrichtung (14) synchron zur Bewegung der Operationstischplatte (2) zu einer dritten Position fahrbar ist.

4. Einrichtung nach Anspruch 3, **dadurch gekennzeichnet, daß** in der dritten Position die Bewegung der Transporteinrichtung (14) mit der angekoppelten Anästhesiestation (3) beendet wird.

5. Einrichtung nach Anspruch 3 oder 4, **dadurch gekennzeichnet, daß** die Lage der Anästhesiestation (3) in der dritten Position unverrückbar gehalten wird.

6. Einrichtung nach einem der Ansprüche 3 bis 5, **dadurch gekennzeichnet, daß** die Bewegung zwischen zweiter und dritter Position nur stattfinden kann, wenn der Aufnahmerahmen (15) sich oberhalb der Untersuchungsstation (7) befindet.

7. Einrichtung nach einem der Ansprüche 1 bis 6, **dadurch gekennzeichnet, daß** der Aufnahmerahmen (15) nur dann vertikal bewegt werden kann, wenn sich Transporteinrichtung (14) in der ersten Position befindet.

8. Einrichtung nach einem der Ansprüche 1 bis 7, **dadurch gekennzeichnet, daß** alternativ zur abnehmbaren Operationstischplatte (2), die Ständer (1) und (8) jeweils mit einer festmontierten Tischplatte ausgestattet sind und der Patient mit Hilfe eines am Aufnahmerahmen (15) befestigbaren Tragetuches zwischen den Tischplatten der Ständer (1) und (8) bewegbar ist .

9. Einrichtung nach Anspruch 1 oder 2, **dadurch gekennzeichnet, daß** die Untersuchungsstation ein Tomograph ist.

10. Einrichtung nach einem der Ansprüche 1 bis 8, **dadurch gekennzeichnet, daß** statt der Untersuchungsstation ein Strahlentherapiegerät (Linearbeschleuniger) vorgesehen ist.

## Claims

1. Arrangement with an operating table top (2) mounted removably on a stand (1), with an anaesthesia station (3), and with an examination station (7) which is arranged at a distance from the operating table and has a stand (8) for receiving the operating table top (2), **characterized by** a ceiling rail (10) which extends over the operating table and the examination station (7), a transport arrangement (14) which is held by the rail (10) and can travel along the latter, and a ceiling mount (5) which supports the anaesthesia station (3), is held by the rail (10) and can travel along the latter, said transport arrangement (14) and said ceiling mount (5) having interacting coupling parts (19, 21) which can be coupled together as soon as the transport arrangement (14) reaches a first position for picking up the operating table top (2).

2. Arrangement according to Claim 1, **characterized in that** the ceiling mount (5) and the transport arrangement (14) coupled to it can be moved to a second position in which the operating table top (2) can be put down onto the receiving stand at the examination station (7).

3. Arrangement according to Claim 1 or 2, **characterized in that** the ceiling mount (5) with the transport arrangement (14) coupled to it can be moved in synchrony with the movement of the operating table top (2) to a third position.

4. Arrangement according to Claim 3, **characterized in that** the movement of the transport arrangement (14) with the coupled anaesthesia station (3) is ended in the third position.

5. Arrangement according to Claim 3 or 4, **characterized in that** the anaesthesia station (3) in the third position is held immovably in place.

6. Arrangement according to one of Claims 3 to 5, **characterized in that** the movement between the second and third positions can take place only when the receiving frame (15) is situated above the examination station (7).

7. Arrangement according to one of Claims 1 to 6, **characterized in that** the receiving frame (15) can be moved vertically only when the transport arrangement (14) is situated in the first position.

8. Arrangement according to one of Claims 1 to 7, **characterized in that**, as an alternative to the removable operating table top (2), the stands (1) and (8) are each equipped with a fixed table top and the patient can be moved between the table tops of the stands (1) and (8) with the aid of a carrying sheet which can be secured to the receiving frame (15).

9. Arrangement according to Claim 1 or 2, **characterized in that** the examination station is a tomograph.

10. Arrangement according to one of Claims 1 to 8, **characterized in that**, instead of the examination station, a radiotherapy apparatus (linear accelerator) is provided.

## Revendications

1. Dispositif comprenant un plateau de table d'opération (2) séparable d'un support (1), un poste d'anesthésie (3) et un poste d'examen (7) situé à distance de la table d'opération et un support (8) pour la réception du plateau de table d'opération (2), **caractérisé en ce qu'**il comporte des rails fixés au plafond (10) au-dessus de la table d'opération et du poste d'examen (7), un dispositif de transport (14) monté mobile sur ces rails (10) et un support plafonnier (5) portant un poste d'anesthésie (3), monté mobile sur ces rails (10), lesquels dispositif de transport (14) et support plafonnier (5) présentent chacun des éléments de couplage interactifs (19, 21) qui peuvent s'accoupler entre eux lorsque le dispositif de transport (14) se trouve dans une première position pour la prise en charge du plateau de table d'opération (2).

2. Dispositif selon la revendication 1 dans lequel le support plafonnier (5) et le dispositif de transport (14) qui lui est accouplé sont mobiles vers une deuxième position dans laquelle le plateau de table d'opération (2) est adaptable sur le support de réception de poste d'examen (7).

3. Dispositif selon la revendication 1 ou 2 dans lequel le support plafonnier (5) et le dispositif de transport (14) qui lui est accouplé sont mobiles vers une troisième position de façon synchrone avec le plateau de table d'opération (2).

4. Dispositif selon la revendication 3 dans lequel le mouvement du dispositif de transport (14) et du poste d'anesthésie (3) accouplés prend fin dans la troisième position.

5. Dispositif selon la revendication 3 dans lequel la position du poste d'anesthésie (3) est maintenue de façon définitive dans la troisième position.

6. Dispositif selon l'une des revendications 3 à 5 dans lequel le mouvement entre la deuxième et la troisième positions ne peut avoir lieu que lorsqu'un cadre de réception (15) du dispositif de transport se trouve au-dessus du poste d'examen (7).

7. Dispositif selon l'une des revendications 1 à 6 dans lequel le cadre de réception (15) ne peut être déplacé verticalement que lorsque le dispositif de transport (14) se trouve dans la première position.

8. Dispositif selon l'une des revendications 1 à 7 dans lequel, alternativement au plateau de table d'opération (2), les supports (1) et (8) sont chacun équipés d'un plateau de table fixe et le patient est transportable entre les plateaux de table des supports (1) et (8) à l'aide d'un drap fixé au cadre de réception (15).

9. Dispositif selon la revendication 1 ou 2 dans lequel le poste d'examen est un tomographe.

10. Dispositif selon l'une des revendications 1 à 8 dans lequel un appareil de radiothérapie (accélérateur linéaire) est prévu à la place du poste d'examen.
